# EUROPEAN PATENT APPLICATION

(11) **EP 2 484 302 A1**
(43) Date of publication of application: **08.08.2012**
(21) Application number: 10153520.1
(22) Date of filing: 08.12.2010
(51) Int. Cl.: A61B 18/14

(54) **Cardiac electrophysiology catheter**

(71) Applicant: Ionescu, Bogdan Gabriel, Bucharest (RO)
(72) Inventor: Ionescu, Bogdan Gabriel, BUCHAREST (RO)

(57) **Abstract**

The invention adds a system to regulate or control electrical impedance/resistance to either pole of cardiac electrophysiology catheters (CEC).

The system is a variable resistor that can be added, for example, to the handle of the catheter itself, for operator comfort of manipulation during the procedure.

The invention applies to any type of CEC.

Cardiac electrophysiology catheters are widely used in the diagnosis and treatment of cardiac arrhythmias and description is available elsewhere.

## Description

The invention refers to a system to regulate or control the electrical impedance/resistance of either pole of cardiac electrophysiology catheters (CEC).

Cardiac electrophysiology procedures are widely used to diagnose and treat cardiac arrhythmias. During such procedures, catheter electrical parameters vary continously and are monitored. The problem consists in the impossibility to control the impedance/resistance of the poles of the catheter during cardiac mapping and ablation.

The present invention introduces a system that allows the operator to control the electrical impedance of the catheter system by adding a variable resistor, that is controllable, to either pole of the catheter. The invention is appliable to either catheter used in cardiac electrophysiology, for cardiac mapping and ablation.

According to the invention, the operator can control the electrical impedance/resistance of the catheter during the procedure, thus turning the procedure more confortable and safer.

## Claims

1. Introduction of a variable electrical resistance to either pole of cardiac electrophysiology catheters (CEC).

2. The system can be added, for example, to the handle of the catheter for operator manipulation during the procedure.

3. Manually or automatic, direct either remote regulation of electrical resistance of either pole of cardiac electrophysiology catheters (CEC).
